# EUROPEAN PATENT SPECIFICATION

(11) **EP 1 496 962 B1**
(45) Date of publication and mention of the grant of the patent: **27.05.2009**
(21) Application number: 03724144.5
(22) Date of filing: 17.04.2003
(51) Int. Cl.: A61M 5/142, A61M 5/168

(54) **IMPLANTABLE THERAPEUTIC SUBSTANCE INFUSION DEVICE WITH MOTOR STALL DETECTOR**
IMPLANTIERBARE INFUSIONSVORRICHTUNG MIT MOTORSTILLSTANDSFESTSTELLUNG
DISPOSITIF DE PERFUSION IMPLANTABLE AVEC DETECTION DE CALAGE DU MOTEUR

(30) Priority: 19.04.2002 US 125858
(43) Date of publication of application: 19.01.2005
(73) Proprietor: MEDTRONIC, INC., Minneapolis MN 55432-5604 (US)
(72) Inventor: SEIFERT, George, P., Shoreview, MN 55126 (US); ROGERS, Charles, R., Maple Grove, MN 55311 (US)
(74) Representative: Hughes, Andrea Michelle
(86) International application number: PCT/US2003/012367
(87) International publication number: WO 2003/089034

(56) References cited:
- WO-A-01/05023
- FR-A- 2 792 840
- GB-A- 2 174 218
- US-A- 5 558 639
- US-B1- 6 264 634

## Description

### FIELD OF THE INVENTION

This disclosure relates to a medical device and more particularly to an implantable therapeutic substance infusion device also known as an implantable drug pump. More particularly, the invention relates to apparatus for detecting drug pump motor stall or missed steps, diagnosing such stalls, and performing corrective actions.

### BACKGROUND OF THE INVENTION

Previously the medical device industry has produced a wide variety of electronic and mechanical devices for treating patient medical conditions. Depending upon medical condition, medical devices can be surgically implanted or connected externally to the patient receiving treatment. Clinicians use medical devices alone or in combination with therapeutic substance therapies and surgery to treat patient medical conditions. For some medical conditions, medical devices provide the best, and sometimes the only, therapy to restore an individual to a more healthful condition and a fuller life. One type of medical device is an implantable therapeutic substance infusion device.

An implantable therapeutic substance infusion device is implanted by a clinician into a patient at a location appropriate for the therapy. Typically, a therapeutic substance infusion catheter is connected to the device outlet and implanted to infuse the therapeutic substance such as a drug or infusate at a programmed infusion rate and predetermined location to treat a condition such as pain, spasticity, cancer, and other medical conditions. Many therapeutic substance infusion devices are configured so the device can be refilled with therapeutic substance through a septum while the device is implanted. Then the time the device can be implanted may not be limited by therapeutic substance stored capacity of the device. An example of an implantable therapeutic substance infusion device is shown in Medtronic, Inc. product brochure entitled "SynchroMed® Infusion System" (1995).

Electrically powered implanted therapeutic substance infusion devices consume energy delivered typically by a battery, also called a power source, and can require replacement once implanted due to depletion of the battery. Typically the most significant power-consuming component in an implantable infusion device is the therapeutic substance metering motor such as a stepper motor.

A stepper motor is an electromechanical device whose rotor rotates a discrete angular amount when an electrical drive pulse is applied to the stator windings. The amplitude and the width of the electrical drive pulse must be tailored to the electromechanical properties of the motor in order to achieve rotation, stability, and optimal energy consumption. An example of instability is the motor rotating backwards or stepping ahead but then "flipping back" to its starting position. For a stepper motor to function normally over a wide power source voltage range, the motor drive pulse needs to be adjusted proportional to the voltage change of the power source.

It is important that an implanted infusion pump infuse the therapeutic substance at the precise rate according to the prescription selected by the attending medical person. Total or intermittent stall of the pump motor will adversely reduce the infusion rate. Since in uncommon but possible circumstances, a pump motor could stall, during which time the drug is not infused as prescribed, it is desirable to detect such stalls and report the event to the attending medical person.

When an implanted infusion pump stalls, the therapeutic substance infusion ceases, and patient does not received the prescribed therapy, it becomes necessary to surgically remove the pump and replace it with another unit. In effect the implanted pump does not achieve its expected service life and must be removed prematurely. This surgery to replace the pump is both unpleasant and expensive. If the pump is not promptly replaced, the patient must be treated in another way, for example using oral medicines. Most importantly some action must be taken when motor stall occurs for the health and welfare of the patient.

An example of related art is US patent 5,550,795 "Electronic Timepiece And A Method of Driving A Stepping Motor of Electronic Timepiece" (August 27, 1996) where an additional detection assisting pulse is supplied to the motor to amplify a voltage induced by the rotational motion which occurs after the main drive pulse is interrupted. A second related art is US patent 5,266,879 "Stepping Motor Driving Circuit" (November 30, 1993) that describes stepping motor drive methods and apparatus. US 6,264,634 and GB-A-2,174,218 also disclose implantable infusion devices with stepper motor monitoring circuitry.

For the foregoing reasons, there is a need for an implantable therapeutic substance infusion device that reliably detects and diagnoses motor stalls.

### BRIEF SUMMARY OF THE INVENTION

A medical device known as an implantable therapeutic substance infusion device is configured for implanting in humans to deliver a therapeutic substance such as pharmaceutical compositions, genetic materials, and biologics to treat a variety of medical conditions such as pain, spasticity, cancer, and many other conditions. The invention provides an implantable infusion device as defined in claim 1. In the preferred embodiment, an apparatus is provided to detect, diagnose, store, and communicate stepper motor stall in an implantable therapeutic substance infusion device. Proper function of this invention improves the safety and cost effectiveness of implantable therapeutic substance pump infusion therapy. Costly clinical procedures are avoided by having this diagnostic measure built into the device. This invention provides further utility in troubleshooting infusion system problems by ruling out the electromechanical system elements. For example, infusion cessation may be due to other system elements such as catheters and fluidic connections.

The implantable infusion device with stepper motor stall detector comprises a housing, a power source, a therapeutic substance reservoir, a therapeutic substance pump, a motor, electronics, a motor drive circuit, a motor measurement circuit, and a motor stall detector. The power source is carried in the housing. The therapeutic substance reservoir is carried in the housing and configured for containing a therapeutic substance and being refilled with the therapeutic substance while implanted. The therapeutic substance pump is carried in the housing and fluidly coupled to the therapeutic substance reservoir and electrically coupled to the power source. The motor is coupled to the power source and coupled to the therapeutic substance pump. The electronics are carried in the housing and coupled to the motor and the power source. The electronics include a processor, memory coupled to the processor, an infusion program residing in memory, the infusion program capable of being modified once the therapeutic substance infusion device is implanted, and transceiver circuitry coupled to the processor for externally receiving and transmitting therapeutic substance infusion device information. The motor drive circuit generates a normal drive pulse to operate the motor and a reverse pulse. The motor coil measurement circuit measures coil normal current during the normal drive pulse and coil reverse current during the reverse pulse. The motor stall detector is configured to compare the coil normal current to the coil reverse current to determine whether the motor is stalled.

### BRIEF DESCRIPTION OF THE DRAWINGS

- FIG. 1: shows the environment of an implantable therapeutic substance infusion device embodiment;
- FIG. 2: shows an implantable therapeutic substance infusion device with catheter embodiment;
- FIG. 3: shows an implantable therapeutic substance infusion device embodiment;
- FIG. 4: shows an exploded view of an implantable therapeutic substance infusion device with peristaltic pump embodiment;
- FIG. 5: shows a schematic block diagram of an implantable therapeutic substance infusion device with major electronic blocks embodiment;
- FIG. 6: shows an electrical drive pulse diagram versus time for the motor control stall detection embodiment;
- FIG. 7A: shows an angular position of the rotor versus time diagram for a successful rotational step embodiment,
- FIG. 7B: shows the motor drive current versus time for a successful rotational step embodiment,
- FIG. 8A: shows an angular position of the rotor versus time diagram for an unsuccessful rotational step embodiment,
- FIG. 8B: shows the motor drive current versus time for an unsuccessful rotational step embodiment,
- FIG. 9A: shows the reverse (or "wrong" polarity) pulse motor drive current embodiment,
- FIG. 9B: shows the superimposed reverse motor drive current with a successful pulse embodiment,
- FIG. 9C: shows the superimposed reverse motor drive current with an unsuccessful pulse embodiment and,
- FIG. 10: shows flow diagram for the stall detection embodiment.

### DETAILED DESCRIPTION OF EXEMPLARY EMBODIMENTS

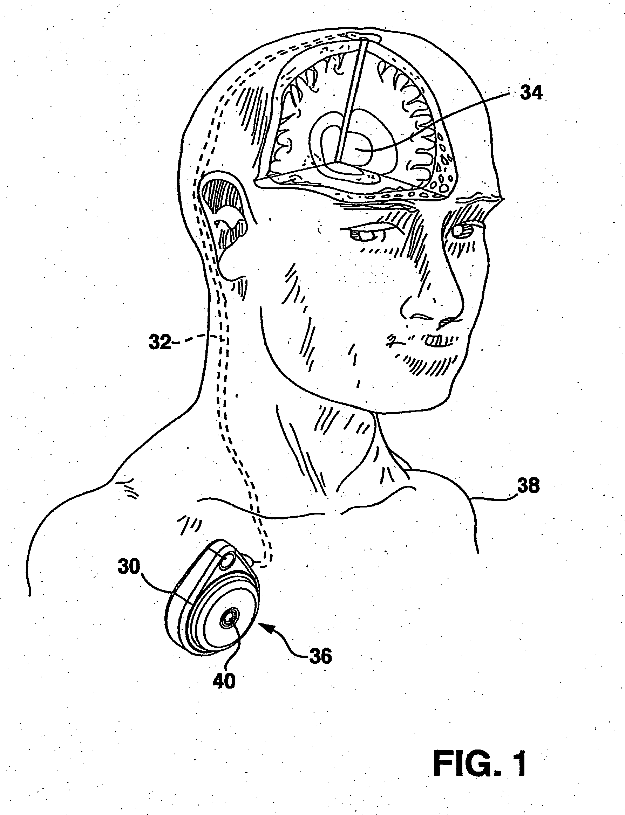

The invention may be embodied in various forms. FIG. 1 shows the environment of an implantable medical device known as an implantable therapeutic substance infusion device 30, also known as a drug pump, having a peristaltic pump embodiment. The therapeutic substance infusion device 30 can be used for a wide variety of therapies such as pain, spasticity, cancer, and many other medical conditions. The implantable therapeutic substance infusion device 30 is typically implanted by a surgeon in a sterile surgical procedure performed under local, regional, or general anesthesia. Before implanting the therapeutic substance infusion device 30, a catheter 32 is typically implanted with the distal end position at the desired therapeutic substance infusion site 34 and the proximal end tunneled to the location where the therapeutic substance infusion device 30 is to be implanted. The implantable therapeutic substance infusion device 30 is generally implanted subcutaneously about 2.5 cm (1.0 inch) beneath the skin where there is sufficient subcutaneous tissue to support the implanted system. Once the therapeutic substance infusion device 30 is implanted into the patient 38, the incision can be sutured closed and the therapeutic substance infusion device 30 can begin operation.

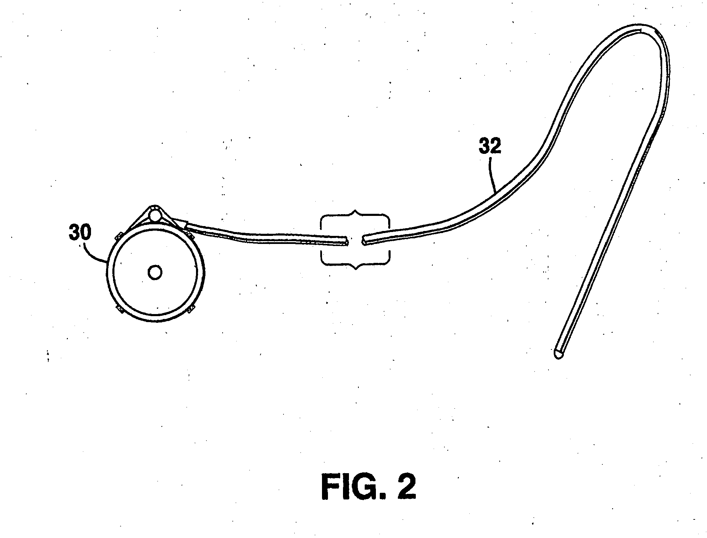

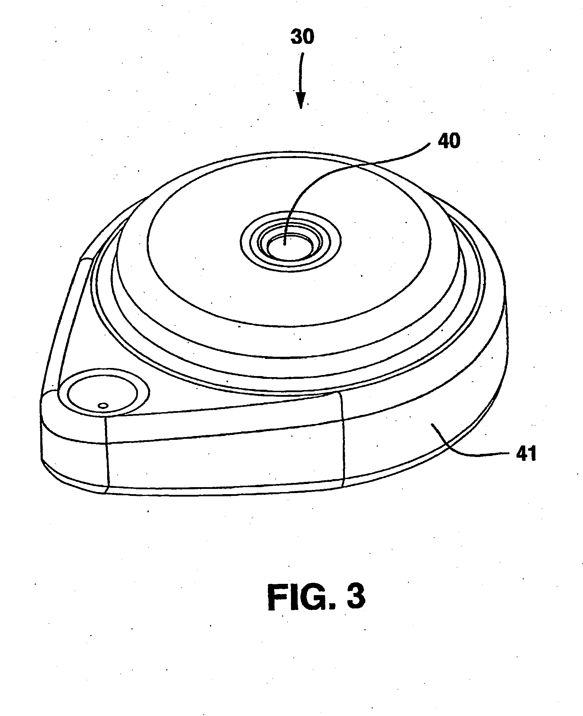

FIG. 2-5 show views of an implantable therapeutic substance infusion device embodiment with pump motor stall detection. The therapeutic substance infusion device 30 operates to infuse a therapeutic substance 36 stored in therapeutic substance reservoir 44 at a programmed rate into a patient 38. The therapeutic substance is a substance intended to have a therapeutic effect such as pharmaceutical compositions, genetic materials, biologics, and other substances. Pharmaceutical compositions are chemical formulations intended to have a therapeutic effect such as intrathecal antispasmodics, pain medications, chemotherapeutic agents, and the like. Pharmaceutical compositions are often configured to function in an implanted environment with characteristics such as stability at body temperature to retain therapeutic qualities, concentration to reduce the frequency of replenishment, and the like. Genetic materials are substances intended to have a direct or indirect genetic therapeutic effect such as genetic vectors, genetic regulator elements, genetic structural elements, DNA, and the like. Biologics are substances that are living matter or derived from living matter intended to have a therapeutic effect such as stem cells, platelets, hormones, biologically produced chemicals, and the like. Other substances are substances intended to have a therapeutic effect yet are not easily classified such as saline solution, fluoroscopy agents, and the like.

The therapeutic substance 36 can be replenished in some embodiments of the implanted therapeutic substance infusion device 30 by inserting a non-coring needle connected to a syringe filled with therapeutic substance 36 through the patient's skin into a septum 40 on the therapeutic substance infusion device 30 to fill the implanted device. The contents of the syringe are then injected into the pump reservoir 44.

If the therapeutic substance infusion device 30 requires replacement due to conditions such as power source depletion or other condition, an incision is made near the implanted therapeutic substance infusion device 30, and the old therapeutic substance infusion device 30 is removed, also known as explanted. After the old therapeutic substance infusion device 30 has been explanted, typically a new therapeutic substance infusion device 30 is then implanted.

The implantable therapeutic substance infusion device with pump motor stall detection comprises a housing 41, a power source 42, a therapeutic substance reservoir 44, a therapeutic substance pump 46, and electronics 48. The housing 41 is manufactured from a material that is biocompatible and hermetically sealed such as titanium, tantalum, stainless steel, plastic, ceramic, and the like. The power source 42 is carried in the housing 41. The power source 42, selected to operate the therapeutic substance pump 46 and electronics 48, may be a lithium ion (Li+) battery, a capacitor, and the like.

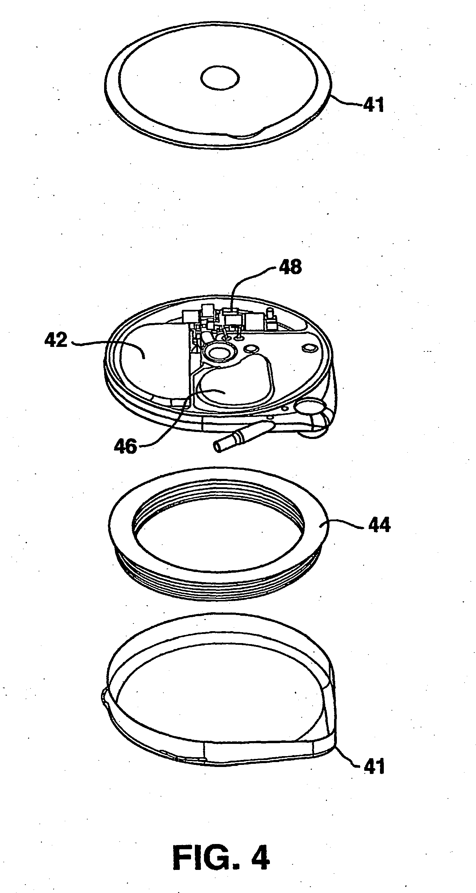

The therapeutic substance reservoir 44 is carried in the housing 41. The therapeutic substance reservoir 44 is configured for containing a therapeutic substance 36. The therapeutic substance reservoir 44 is configured to be refilled with therapeutic substance 36 while implanted. The therapeutic substance pump 46 is carried in the housing 41. The therapeutic substance pump assembly 46 is fluidly coupled to the therapeutic substance reservoir 44 and electrically coupled to the power source 42. The therapeutic substance pump 46 is a pump sufficient for infusing therapeutic substance 36 such as the peristaltic pump with stepper motor drive (FIG. 4) that can be found in the SynchroMed® Infusion System available from Medtronic, Inc. A stepper motor is an electromechanical device whose rotor rotates a discrete angular amount when an electrical drive pulse is applied to the stator windings. The amplitude and the width of the pulse must be tailored to the electromechanical properties of the motor in order to achieve rotation, rotational stability, and optimal energy consumption. An example is a motor that rotates 180 degrees with the application of a 3 volt, 11.2 millisecond, square pulse. A second pulse is then applied at minus 3 volts to rotate an additional 180 degrees making a complete revolution of the rotor. If a pulse is applied in the same polarity as the previous rotational pulse, referred to here as a reverse pulse, the rotor does not step but is locked or artificially stalled in place. The electromechanical properties of the motor are such that rotation can only occur in one direction.

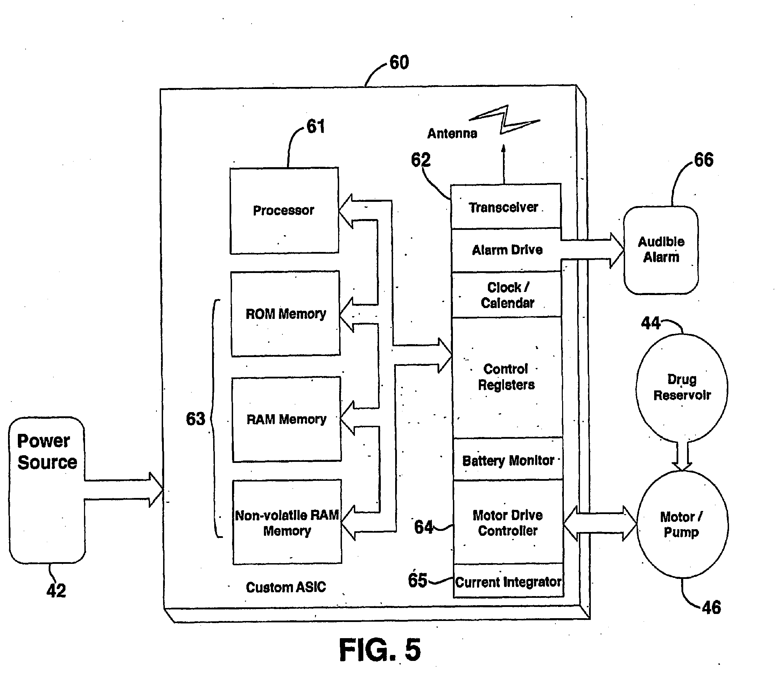

FIG. 4 shows electronics 48 carried in the housing 41. FIG. 5 shows a pump block diagram where the electronic block diagram 60 is coupled to the motor 46, the power source 42 and the audible alarm 66. The electronic block diagram 60 includes a processor, memory 63 coupled to the processor 61, control registers, motor drive controller 64, integrator circuit 65, and other functional components. An infusion program resides in memory 63, the infusion program capable of being modified once the therapeutic substance infusion device is implanted. Transceiver circuitry 62 is coupled to the processor for externally receiving and transmitting therapeutic substance infusion device information. All pump diagnostic and performance data obtained or stored in memory 63, including data obtained and stored for stall detection, can be transmitted outside the pump by the transceiver 62 to a medical person for their use.

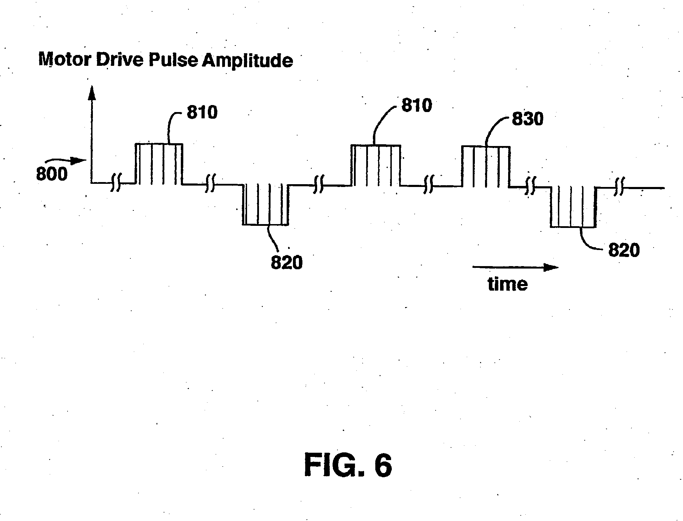

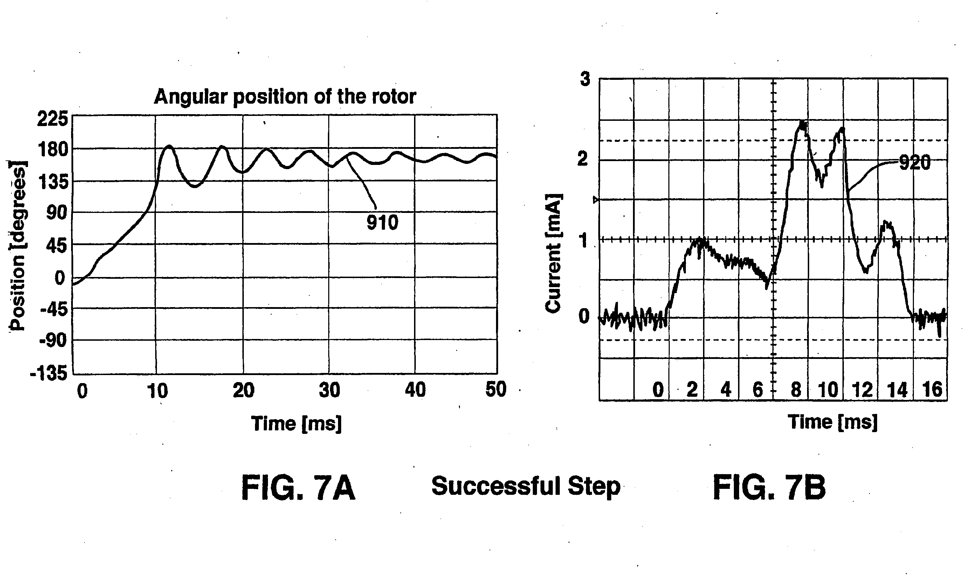

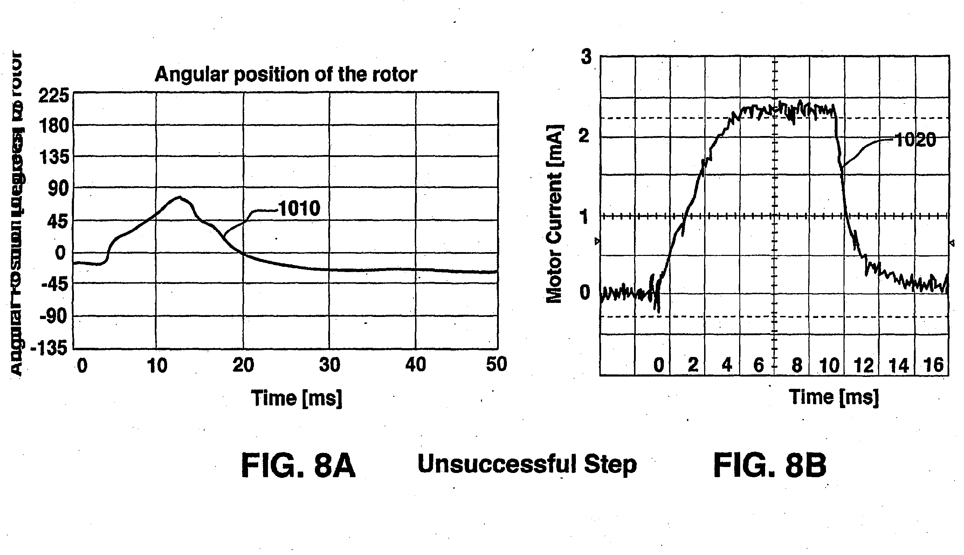

FIG. 6 shows periodic normal drive pulses 810 and 820, and a periodic reverse drive pulse 830, all pulses generated by the motor drive controller 64 to drive the motor 46. FIG. 7A shows the angular rotation 910 in response to a successful drive pulse 920 in FIG. 7B. This is the desired functionality. For normal motor function the intent is that each motor pulse is successful and no unsuccessful pulses occur. FIG. 8A shows that the angular position 1010 was unchanged due to an unsuccessful motor drive pulse of FIG. 8B. This is an undesirable result. An unsuccessful motor drive pulse is equivalent to motor stall.

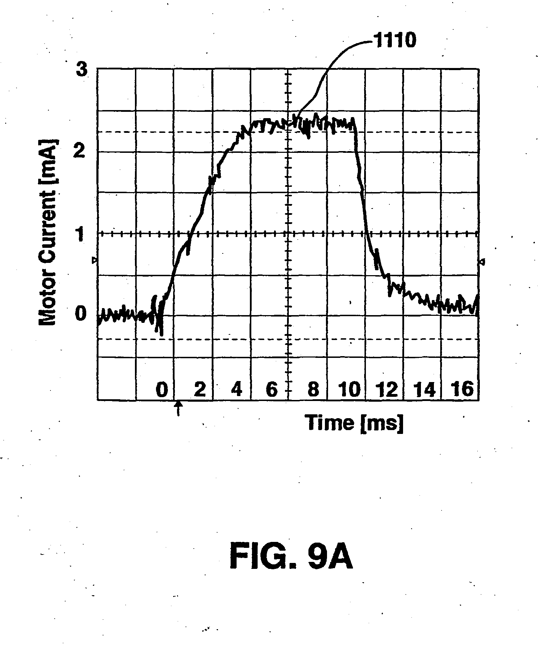

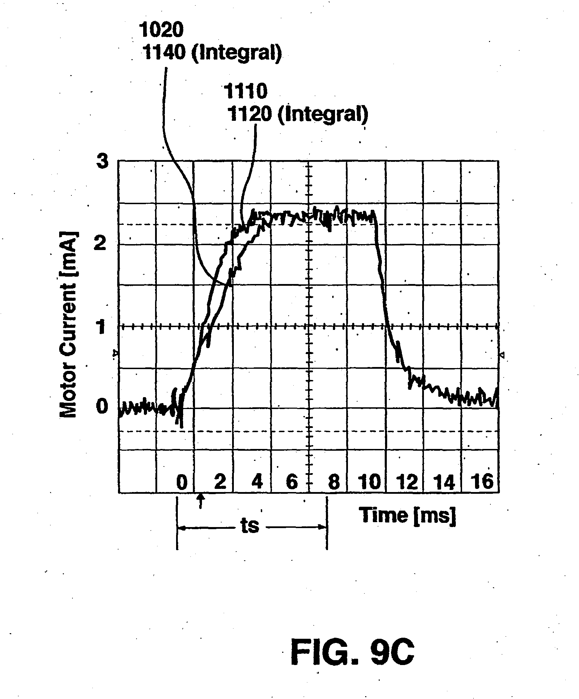

FIG. 9A illustrates the motor drive current pulse 1110 due to reverse motor drive pulse 830 in FIG. 6. The reverse motor drive pulse current 1110 has been shown to be substantially equivalent to an unsuccessful current pulse 1020 in FIG. 8B, and this is illustrated in FIG. 9C where pulses 1020 and 1110 are about equal. Therefore, the reverse motor drive pulse 1110 can be used to determine if a stall condition is detected.

The motor drive current integrator 65 measures and integrates the coil current 920 in FIG. 7B during a normal successful drive pulse and coil reverse current 1110 of FIG. 9A during a reverse drive pulse. The motor drive current integrator 65 also measures and integrates coil current 1020 in FIG. 8B during an unsuccessful drive pulse. The processor 61 makes several computations based on these stored motor current integration data, and goes through a decision algorithm to invoke various actions and stores the results described below.

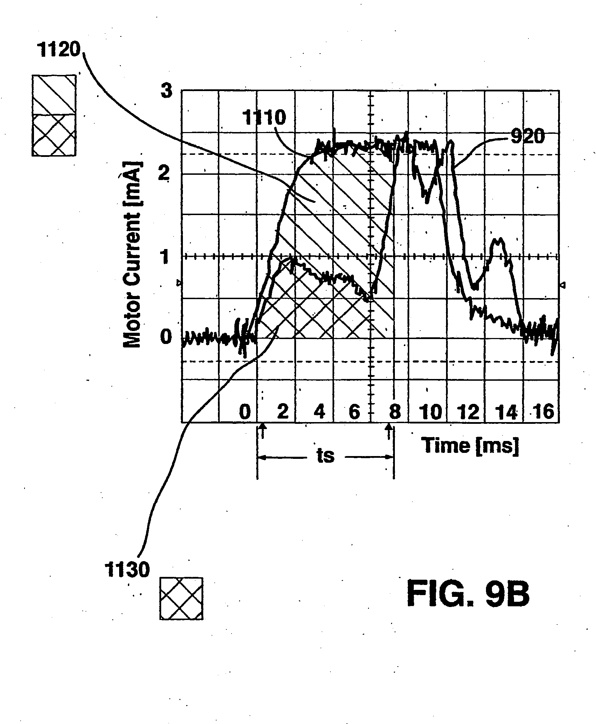

The normal successful step motor current 920 in FIG. 7B versus time is reproduced in FIG.9B. Similarly, the reverse step motor current pulse 1110 in FIG. 9A versus time is reproduced in FIG.9B. The integrated successful coil currents up to time tₛ 1130 is compared to the integrated reverse coil current 1120. A portion of the drive pulse for integration, preferably the first one half to two thirds, is selected based upon motor characteristics so that the selected portion of the drive pulse occurs approximately prior to the rotor completing its turn. This selection maximizes the difference in integrated currents 1130 and 1120. The integrations generate digital numbers that are inserted in the motor stall inequality equation *STALLINTₙₒᵣₘₐₗ ≥ STALLINTᵣₑᵥₑᵣₛₑ -Offset,* where *STALLINTₙₒᵣₘₐₗ* 1130 or 1140 is the integrated normal coil current or stall coil current, *STALLINTᵣₑᵥₑᵣₛₑ* 1120 is the integrated reverse coil current, and *Offset* is zero or a positive number to decrease or offset the value of *STALLINTᵣₑᵥₑᵣₛₑ. Offset* is used to accommodate for small, substantially fixed differences between integrated reverse current 1120 and integrated stall current 1140. If the inequality in this stall detection equation is true then the motor is determined to be stalled. FIG. 9B shows that for a non-stalled integrated normal current 1130 the inequality will be false by comparison to integrated reverse current 1120. FIG. 9C shows that for a stalled integrated normal current 1140 the inequality will be true by comparison to integrated reverse current 1120.

This stall detection equation reliably detects stall because the integrated motor coil currents are quite dependent on whether the motor rotor rotates or does not rotate normally. In addition, since the integrated reverse coil current is substantially the same as the integrated stall coil current, the algorithm has a continuous value for the integrated current if a stall were to occur.

In order to increase the accuracy of the stall determination, at least one more motor stall inequality equation is calculated on a sequential normal drive pulse and reverse pulse to validate that the motor is stalled. The sequential reverse pulse provides a real-time calibration reading as input to the algorithm.

After a stall is determined it may be possible to overcome the stall by significantly increasing the motor drive pulse energy. After higher motor drive energy is applied, the stall test described above can be repeated to determine if the stall was overcome. After a stall is overcome, eventually the motor drive pulse energy could revert to the normal level. There are many sequences of testing and pulse energy manipulation possible in order to overcome stall and confirm normal function. Motor energy may be increased by manipulating voltage, current, pulse width, drive duty cycles, and the like.

An additional feature of the integrated coil motor current is that if the integration value is at or near zero then the motor coil is determined to be an open circuit. An open circuit coil would cause a motor stall. If an open coil is detected, valuable diagnostic information about the pump is obtained to differentiate from other causes of motor stall or other potential system issues such as those relating to catheters and fluidic connections.

Another feature of the integrated reverse coil current measurement is to provide a continuous automatic calibration of the system. For example, the integrated reverse coil current provides calibration to compare to integrated normal coil current to continuously distinguish successful steps from unsuccessful steps. The integrated coil current encompasses many variables including power source depletion, motor torque variation due to mechanical wear, corrosion, roller position, and motor speed, and voltage drive variation due to operated alarms and bolus infusion. By taking periodic reverse readings the algorithm can account for the present electromechanical status.

In order to minimize the power source energy consumption and maximize the infusion accuracy, the reverse pulse 830 resulting in current pulse 1110 is delivered at a lower frequency than the normal drive pulses, preferably less than or equal to one in every 500 drive pulses. This ratio of reverse to normal drive pulses accounts for a potential 0.2% infusion error that, if necessary, can be accounted for in the calibration constant for each pump.

An additional capability of the preferred embodiment is a zero drive pulse. Zero drive simply means measuring the integrated current over the typical sampling interval without a motor normal drive pulse present. The resulting value may be greater than zero due to circuit offsets or system noise. The integrated zero pulse measurement is compared to the integrated normal coil current to determine whether the motor coil is open circuit. The zero pulse measurement can be used in conjunction with the reverse pulse measurement to further refine the stall detection accuracy.

Similar to FIG. 9B, the integrated normal coil current can be compared to the integrated zero coil current using an open coil inequality equation: *STALLINTₙₒᵣₘₐₗ ≤ STALLINT_{zero} + Offset ,* where *STALLINTₙₒᵣₘₐₗ* is the integrated normal coil current 1130, *STALLINT_{zero}* is integrated zero coil current, and *Offset* is zero or a positive number to increase or offset the value of *STALLINT_{zero}*.

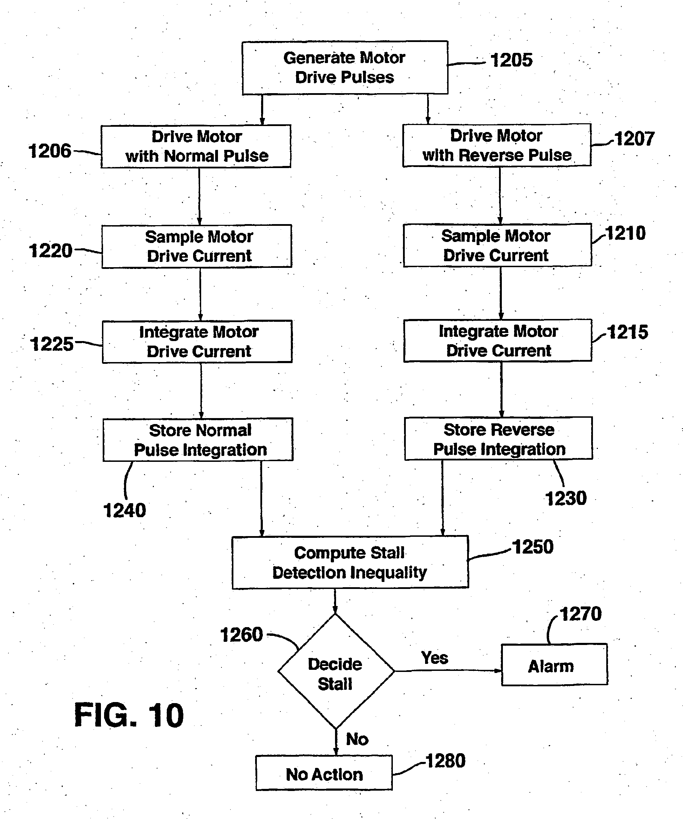

FIG. 10 illustrates a flow diagram of motor stall detection. First the motor drive pulses 800 are generated 1205. The motor is driven with a normal pulse 1206 and shortly thereafter a reverse pulse 1207. Both resultant current pulses are sampled 1220 and 1210, and then integrated 1225 and 1215. The measured integrated motor current pulses are stored 1240 and 1230 in registers to be retrieved for the stall computation 1250. To determine if a motor stall condition exists, both integrated currents are compared 1250 using the stall detection equations described above. If a stall is detected due to an unsuccessful step, an alarm 1270 in the infusion device may be actuated so the patient or caregiver is made aware of the stall condition. If no stall is detected, no further action 1280 is taken. This flow diagram is one of many that could be constructed to describe the operation of various elements of this invention.

Typical implantable battery powered therapeutic substance infusion devices have the capability of storing and transmitting data to an external receiver system for use by an attending medical person. Similarly, all motor drive pulse current, derived decisions, occurrence date and the like data such as described in this invention may be stored in the device and transmitted.

Any implantable battery powered therapeutic substance infusion device with any type of substance pumping electromechanical mechanism could be configure to measure the normal drive electrical parameters and comparing these parameters to similar subnormal drive parameters to detect non-normal function.

One skilled in the art will appreciate that the present invention can be practiced with embodiments other than those disclosed. The disclosed embodiments are presented for purposes of illustration and not limitation, and the present invention is limited only by the claims that follow.

## Claims

1. An implantable infusion device (30) with stepper motor stall detector, comprising:
a housing (41);
a power source (42) carried in the housing (41);
a therapeutic substance reservoir (44) carried in the housing (41), the therapeutic substance reservoir (44) configured for containing a therapeutic substance (36) and being refilled with the therapeutic substance (36) while implanted;
a therapeutic substance pump (46) carried in the housing (41), the therapeutic substance pump (46) fluidly coupled to the therapeutic substance reservoir (44), and electrically coupled to the power source (42);
a motor coupled to the power source (42) and coupled to the therapeutic substance pump (46); electronics (48) carried in the housing (41), the electronics (48) coupled to the motor and the power source (42), the electronics (48) including,
a processor(61),
memory (63) coupled to the processor (61), an infusion program residing in memory (63), the infusion program capable of being modified once the therapeutic substance infusion device (30) is implanted,
transceiver circuitry (62);
a motor drive circuit that generates a normal drive pulse to operate the motor (46), and a reverse pulse;
a motor coil measurement circuit to measure coil normal current during the normal drive pulse, coil reverse current during the reverse pulse; and, a motor stall detector configured to compare the coil normal current to the coil reverse current to determine whether the motor is stalled; **characterized in that** said transceiver circuitry (62) is coupled to the processor (61) for externally receiving and transmitting therapeutic substance infusion device information, wherein the motor stall detector comprises means for providing an integral of measured coil normal current values and an integral of measured reverse current values, and means for comparing the coil normal current integral to the coil reverse current integral using a motor stall inequality equation *STALLINTₙₒᵣₘₐₗ* ≥ *STALLINTᵣₑᵥₑᵣₛₑ - Offset*
where *STALLINTₙₒᵣₘₐₗ* is coil normal current integral,
*STALLINTᵣₑᵥₑᵣₛₑ* is coil reverse current integral, and
*Offset* is zero or a positive number to decrease or offset the value of
*STALLINTᵣₑᵥₑᵣₛₑ;* and wherein said motor stall detector is adapted to determine the motor to be stalled when the inequality equation is true.

2. The implantable infusion device (30) as in claim 1 wherein when the motor stall detector is adapted to calculate at least one more motor stall inequality equation on a subsequent normal drive pulse and reverse pulse to validate that the motor is stalled, on determination that the motor is stalled.

3. The implantable infusion device (30) as in claim 1 wherein when the coil motor current integral is near zero then the motor coil is open.

4. The implantable infusion device (30) as in claim 1 wherein the coil normal current integral is taken from a selected portion of the normal drive pulse.

5. The implantable infusion device (30) as in claim 4 wherein the selected portion of the normal drive pulse is selected based upon motor characteristics, so the selected portion of the normal drive pulse occurs when a rotor is turning.

6. The implantable infusion device (30) as in claim 1 wherein the coil reverse current integral provides calibration for coil normal current integrated under a stall condition.

7. The implantable device (30) as in claim 1 wherein motor data from the motor coil measurement circuit and the motor stall detector are stored in memory and transmitted by the transceiver circuitry.

8. The implantable infusion device (30) as in claim 1 wherein the reverse pulse is delivered at a lower frequency than a normal drive pulse.

9. The implantable infusion device (30) as in claim 8 wherein the reverse pulse is delivered about every 500 drive pulses.

10. The implantable device (30) as in claim 1 wherein when the motor is determined to be stalled a motor drive circuit generates a normal drive pulse with a larger pulse width in an attempt to restart the motor:

11. The implantable device (30) as in claim 10 wherein when the motor restarts, the motor drive circuit generates a normal drive pulse.

12. The implantable infusion device (30) as in claim 1 wherein the coil reverse current integral is performed on a reverse pulse.

13. The implantable infusion device (30) as in claim 1 wherein at least one reverse pulse is delivered once a motor stall is detected to specifically calibrate coil normal current integral for a stall condition.

14. The implantable infusion device (30) as in claim 1 further comprising a zero drive pulse generated by the motor drive circuit that is measured by the motor coil measurement circuit as a coil zero current integral and compared to the coil normal current integral to determine whether the motor coil is open.

## Patentansprüche

1. Implantierbare Infusionsvorrichtung (30) mit einer Schrittmotorstillstandserkennung, welche aufweist:
ein Gehäuse (41),
eine Energiequelle (42), die in dem Gehäuse (41) aufgenommen ist,
ein Reservoir (44) für eine therapeutische Substanz, das in dem Gehäuse (41) aufgenommen ist, wobei das Reservoir (44) für die therapeutische Substanz eingerichtet ist, um eine therapeutische Substanz (36) zu enthalten und mit der therapeutischen Substanz (36) nachgefüllt zu werden, während es implantiert ist,
eine Pumpe (46) für die therapeutische Substanz, die in dem Gehäuse (41) aufgenommen bzw. getragen ist, wobei die Pumpe (46) für die therapeutische Substanz in Fluidverbindung mit dem Reservoir (44) für die therapeutische Substanz steht und elektrisch mit der Energiequelle (42) verbunden ist,
einen Motor, der mit der Energiequelle (42) und mit der Pumpe (46) für die therapeutische Substanz verbunden ist, eine Elektronik (48), die in dem Gehäuse (41) aufgenommen ist, wobei die Elektronik (48) mit dem Motor und der Energiequelle (42) verbunden ist, wobei die Elektronik (48) aufweist
einen Prozessor (61),
Speicher (63), der mit dem Prozessor (61) verbunden ist, ein Infusionsprogramm, das in dem Speicher (63) abgelegt ist, wobei das Infusionsprogramm in der Lage ist, verändert zu werden, sobald die Infusionsvorrichtung (30) für die therapeutische Substanz implantiert ist,
eine Transceiverschaltung (62),
eine Motorantriebsschaltung, die einen normalen Antriebsimpuls erzeugt, um den Motor (46) zu betreiben, und einen Umkehrimpuls,
eine Motorspulen- bzw. -wicklungsmessschaltung, um den Spulennormalstrom während des normalen Antriebsimpulses und den Spulenumkehrstrom während des Umkehrimpulses zu messen, und eine Motorstillstandserkennung, die dazu eingerichtet ist, den Motornormalstrom mit dem Motorumkehrstrom zu vergleichen, um zu bestimmen, ob der Motor blockiert ist, **dadurch gekennzeichnet, dass** die Transceiverschaltung (62) mit dem Prozessor (61) zum externen Empfangen und Senden von Informationen über die Infusionsvorrichtung für die therapeutische Substanz verbunden ist, wobei die Motorstillstandserkennung Mittel zum Bereitstellen eines Integrals der gemessenen Spulenormalstromwerte und eines Integrals der gemessenen Umkehrstromwerte aufweist und Mittel zum Vergleichen des Spulennormalstromintegrals mit dem Spulenumkehrstromintegral unter Verwendung einer Motorstillstandsungleichung *STAL-LINTₙₒᵣₘₐₗ ≥ STALLINTᵣₑᵥₑᵣₛₑ - Offset,*
wobei *STALLINTₙₒᵣₘₐₗ* das Spulennormalstromintegral ist,
*STALLINTᵣₑᵥₑᵣₛₑ* das Spulenumkehrstromintegral ist und
*Offset* Null oder eine positive Zahl ist, um den Wert von *STALLINTᵣₑᵥₑᵣₛₑ* zu verringern oder zu verschieben, und wobei die Motorstillstandserkennung dazu eingerichtet ist, zu erkennen, dass der Motor blockiert ist, wenn die Ungleichung wahr ist.

2. Implantierbare Infusionsvorrichtung (30) nach Anspruch 1, wobei, wenn die Motorstillstandserkennung dazu eingerichtet ist, wenigstens eine weitere Motorstillstandsungleichung auf einen nachfolgenden Normalantriebsimpuls und Umkehrimpuls zu berechnen, um zu bestätigen, dass der Motor blockiert ist, auf die Bestimmung hin, dass der Motor blockiert ist.

3. Implantierbare Infusionsvorrichtung (30) nach Anspruch 1, bei der die Motorspule offen ist, wenn das Spulenmotorintegral nahezu bzw. im wesentlichen null ist.

4. Implantierbare Infusionsvorrichtung (30) nach Anspruch 1, bei der das Spulennormalstromintegral über einen ausgewählten Anteil des Normalantriebsimpulses berechnet wird.

5. Implantierbare Infusionsvorrichtung (30) nach Anspruch 4, bei der der ausgewählte Anteil des Normalantriebsimpulses basierend auf Motorcharakteristiken ausgewählt wird, so dass der ausgewählte Anteil des Normalantriebsimpulses auftritt, wenn sich ein Rotor dreht.

6. Implantierbare Infusionsvorrichtung (30) nach Anspruch 1, bei der das Spulenumkehrstromintegral eine Kalibrierung für den unter einer Blockadebedingung integrierten Spulennormalstrom bereitstellt.

7. Implantierbare Vorrichtung (30) nach Anspruch 1, bei der die Motordaten von der Motorspulenstrommessschaltung und der Motorstillstandserkennung im Speicher gespeichert und mittels der Transceiverschaltung übertragen werden.

8. Implantierbare Infusionsvorrichtung (30) nach Anspruch 1, bei der der Umkehrimpuls mit einer niedrigeren Frequenz als ein Normalantriebsimpuls abgegeben wird.

9. Implantierbare Infusionsvorrichtung (30) nach Anspruch 8, bei der der Umkehrimpuls ungefähr alle 500 Antriebsimpulse abgegeben wird.

10. Implantierbare Vorrichtung (30) nach Anspruch 1, bei der eine Motorantriebsschaltung einen Normalantriebsimpuls mit einer größeren Pulsbreite erzeugt, um den Motor neu zu starten, wenn bestimmt worden ist, dass der Motor blockiert ist.

11. Implantierbare Vorrichtung (30) nach Anspruch 10, bei der die Motorantriebsschaltung einen Normalantriebsimpuls erzeugt, wenn der Motor neu startet.

12. Implantierbare Infusionsvorrichtung (30) nach Anspruch 1, bei der das Spulenumkehrstromintegral über einen Umkehrpuls berechnet wird.

13. Implantierbare Infusionsvorrichtung (30) nach Anspruch 1, bei der wenigstens eine Umkehrpuls abgegeben wird, sobald ein Motorstillstand erkannt wird, um das Spulennormalstromintegral für eine Stillstandsbedingung spezifisch zu kalibrieren.

14. Implantierbare Infusionsvorrichtung (30) nach Anspruch 1, ferner umfassend einen Nullantriebsimpuls, der von der Motorantriebsschaltung erzeugt und von der Motorspulenmessschaltung als ein Spulennullstromintegral gemessen und mit dem Spulennormalstromintegral verglichen wird, um zu bestimmen, ob die Motorspule offen ist.

## Revendications

1. Dispositif de perfusion implantable (30) ayant un détecteur de calage de moteur pas-à-pas, comportant :
un logement (41) ;
une source d'alimentation (42) contenue dans le logement (41);
un réservoir de substance thérapeutique (44) contenu dans le logement (41), le réservoir de substance thérapeutique (44) étant configuré pour contenir une substance thérapeutique (36) et étant à nouveau rempli de la substance thérapeutique (36) lorsque implanté ;
une pompe de substance thérapeutique (46) contenue dans le logement (41), la pompe de substance thérapeutique (46) étant couplée de manière fluide au réservoir de substance thérapeutique (44), et électriquement couplée à la source d'alimentation (42) ;
un moteur couplé à la source d'alimentation (42) et couplé à la pompe de substance thérapeutique (46) ; un composant électronique (48) contenu dans le logement (41), le composant électronique (48) étant couplé au moteur et à la source d'alimentation (42), le composant électronique (48) incluant,
un processeur (61),
une mémoire (63) couplée au processeur (61), un programme de perfusion résidant en mémoire (63), le programme de perfusion pouvant être modifié une fois que le dispositif de perfusion de substance thérapeutique (30) est implanté,
un circuit d'émission-réception (62) ;
un circuit d'entrainement de moteur qui génère une impulsion d'entrainement normale pour mettre en fonctionnement le moteur (46), et une impulsion inverse ;
un circuit de mesure de bobine du moteur pour mesurer un courant normal de bobine pendant l'impulsion d'entraînement normale, un courant inverse de bobine pendant l'impulsion inverse ; et un détecteur de calage du moteur configuré pour comparer le courant normal de bobine au courant inverse de bobine afin de déterminer si le moteur a calé ; **caractérisé en ce que** ledit circuit d'émission-réception (62) est couplé au processeur (61) pour recevoir et transmettre en externe des informations du dispositif de perfusion de substance thérapeutique, dans lequel le détecteur de calage du moteur comporte des moyens pour fournir une intégrale des valeurs du courant normal de bobine mesuré et
une intégrale des valeurs du courant inverse mesuré, et des moyens pour comparer l'intégrale du courant normal de bobine à l'intégrale du courant inverse de bobine en utilisant une équation d'inégalité de calage de moteur
*STALLINTₙₒᵣₘₐₗ ≥ STALLINTᵣₑᵥₑᵣₛₑ - Offset*
où *STALLINTₙₒᵣₘₐₗ* est l'intégrale du courant normal de bobine,
*STALLINTᵣₑᵥₑᵣₛₑ* est l'intégrale du courant inverse de bobine, et
*Offset* est un nombre égal à zéro ou positif pour réduire ou décaler la valeur de *STALLINTᵣₑᵥₑᵣₛₑ ;* et dans lequel ledit détecteur de calage du moteur est adapté pour déterminer que le moteur a calé lorsque l'équation d'inégalité est vraie.

2. Dispositif de perfusion implantable (30) selon la revendication 1 dans lequel lorsque le détecteur de calage du moteur est adapté pour calculer au moins une équation d'inégalité de calage du moteur supplémentaire sur une impulsion d'entrainement normale et une impulsion inverse ultérieures afin de valider que le moteur a calé, lorsqu'il est déterminé que le moteur a calé.

3. Dispositif de perfusion implantable (30) selon la revendication 1 dans lequel l'intégrale du courant de moteur de la bobine est proche de zéro lorsque la bobine du moteur est ouverte.

4. Dispositif de perfusion implantable (30) selon la revendication 1 dans lequel l'intégrale du courant normale de la bobine est extraite d'une partie sélectionnée de l'impulsion d'entrainement normale.

5. Dispositif de perfusion implantable (30) selon la revendication 1 dans lequel la partie sélectionnée de l'impulsion d'entrainement normale est sélectionnée sur la base des caractéristiques du moteur, la partie sélectionnée de l'impulsion d'entrainement normale apparaît donc lorsqu'un rotor tourne.

6. Dispositif de perfusion implantable (30) selon la revendication 1 dans lequel l'intégrale de courant inverse de la bobine fournit le calibrage pour le courant normal de la bobine intégré dans une condition de calage.

7. Dispositif de perfusion implantable (30) selon la revendication 1 dans lequel des données du moteur provenant du circuit de mesure de la bobine du moteur et du détecteur de calage du moteur sont mémorisées en mémoire et transmises par le circuit d'émission-réception.

8. Dispositif de perfusion implantable (30) selon la revendication 1 dans lequel l'impulsion inverse est délivrée à une fréquence inférieure à celle d'une impulsion d'entrainement normale.

9. Dispositif de perfusion implantable (30) selon la revendication 8 dans lequel l'impulsion inverse est délivrée environ toutes les 500 impulsions d'entrainement.

10. Dispositif de perfusion implantable (30) selon la revendication 1 dans lequel lorsque le moteur est déterminé comme ayant calé un circuit d'entrainement du moteur génère une impulsion d'entrainement normale avec une largeur d'impression plus grande lors d'une tentative de redémarrage du moteur.

11. Dispositif de perfusion implantable (30) selon la revendication 10 dans lequel lorsque le moteur redémarre, le circuit d'entrainement du moteur génère une impulsion d'entrainement normale.

12. Dispositif de perfusion implantable (30) selon la revendication 1 dans lequel l'intégrale du courant inverse de la bobine est exécutée sur une impulsion inverse.

13. Dispositif de perfusion implantable (30) selon la revendication 1 dans lequel au moins une impulsion inverse est délivrée une fois qu'un calage du moteur est détecté pour calibrer de manière spécifique l'intégrale du courant normal de la bobine pour une condition de calage.

14. Dispositif de perfusion implantable (30) selon la revendication 1 comportant en outre une impulsion d'entrainement nulle générée par le circuit d'entrainement du moteur qui est mesurée par le circuit de mesure de la bobine du moteur en tant qu'intégrale du courant nul de la bobine et comparée à l'intégrale du courant normale de la bobine pour déterminer si la bobine du moteur est ouverte.
